# FASCICULE DE BREVET EUROPEEN

(11) **EP 1 198 995 B1**
(45) Date de publication et mention de la délivrance du brevet: **27.04.2005**
(21) Numéro de dépôt: 01440050.1
(22) Date de dépôt: 27.02.2001
(51) Int. Cl.: A23L 1/30, A23L 1/305, A61K 31/20, A23G 1/00

(54) **Composition anti-stress destinée à être incorporée principalement à des véhicules nutritionnels**
Anti-Stress Zusammensetzung zum Einbringen in essbaren Träger
Anti-stress composition for incorporation in nutritional carriers

(30) Priorité: 17.10.2000 FR 0013298
(43) Date de publication de la demande: 24.04.2002
(73) Titulaire: Laboratoires Robert Schwartz, 67400 Illkirch Graffenstaden (FR)
(72) Inventeur: Schwartz, Robert, 75016 Paris (FR); Chantereau, Sylvie, 67610 La Wantzenau (FR); Anton, Jean-Christophe, 67000 Strasbourg (FR)
(74) Mandataire: Littolff, Denis

(56) Documents cités:
- EP-A- 0 896 797
- DE-A- 4 029 549
- US-A- 4 621 137
- US-A- 5 922 704
- SNYDER H.E., KWON T.W.: "Soybean Utilization" 1987 , VAN NOSTRAND REINHOLD , NEW YORK, US XP002172484 146060 * page 33 - page 34 * * page 64 - page 66 * * page 45; tableau 2.6 *
- PATENT ABSTRACTS OF JAPAN vol. 004, no. 093 (C-017), 5 juillet 1980 (1980-07-05) & JP 55 054883 A (ASAMA KASEI KK), 22 avril 1980 (1980-04-22)
- DATABASE MEDLINE [en ligne] AN-2000227059, 7 juillet 2000 (2000-07-07) YEHUDA ET AL.: "Fatty acid mixture counters stress changes in cortisol, cholesterol and impair learning" XP002172485 & INTERNATIONAL JOURNAL OF NEUROSCIENCE., vol. 101, no. 1-4, 2000, pages 73-87, GORDON AND BREACH., US ISSN: 0020-7454

## Description

La présente invention concerne une composition en vue d'obtenir un triple effet calmant, régulateur des hormones du stress et immuno-stimulateur.

Il est maintenant scientifiquement établi que le stress se développe selon les trois phases suivantes :
- dans un premier temps, le corps réagit à une situation extérieure d'agression par une réaction d'alarme qui est manifestée par plusieurs signes physiques, dont principalement une accélération du rythme cardiaque, une respiration courte et rapide, l'augmentation générale de la tension musculaire et une sécheresse de la gorge. Le but de cette réaction est d'améliorer l'oxygénation des tissus et de répartir le sang de manière différente dans l'organisme, en particulier vers sa périphérie. Ces mécanismes, mis en action par le corps, confèrent en principe à l'individu des dispositions physiques lui permettant de lutter contre l'agression extérieure ;
- dans un second temps, la résistance peut s'organiser: l'organisme est en principe prévu pour s'adapter à l'agression, puisqu'il dispose de tous les nutriments (oxygène, glucose, libération d'adénosine triphosphate) lui permettant de résister sur une période courte ou de moyenne durée à la situation vécue. Il met en fait en oeuvre ce qu'il est convenu d'appeler une "stratégie de survie". Les mécanismes qui sont mis à contribution par le corps peuvent cependant être soit en adéquation, soit en inadéquation avec la réponse optimale à apporter. Pendant cette phase, les performances intellectuelles diminuent, et on observe chez le patient une tendance à déprimer, voire à abuser de l'alcool ou d'autres drogues ;
- vient ensuite le stade d'épuisement: les signes physiques sont une tension musculaire très forte, avec des maux de tête et des migraines fréquentes, et un transit intestinal perturbé (spasmes et douleurs). Dans certains cas, on note l'apparition d'ulcères et, d'une manière générale, il existe un effet de renforcement des pathologies sous-jacentes. A ce stade, le syndrome dépressif est franc.

Au cours de ces trois phases, le système nerveux intervient par une sécrétion de différentes hormones. Les plus connues sont l'adrénaline (sécrétée lors de la réaction initiale d'alarme), le cortisol et la corticostérone. D'autres monoamines sont également synthétisées et libérées. L'augmentation de ces hormones suite à une situation de stress donne à l'organisme les moyens de sa défense en stockant de l'énergie, en mobilisant des réserves et en le désensibilisant à certains types d'agressions tels que la douleur. A moyen et long terme cependant, les effets de ces hormones sont souvent néfastes, voire désastreux. Elles provoquent un épuisement de l'organisme, le rendent vulnérable aux infections (diminution des défenses immunitaires) et altèrent les neurones (pertes de mémoire). Ces effets ont enfin un impact entre autres sur le système cardiovasculaire, le vieillissement de l'organisme et certaines conditions d'apparition d'un cancer.

Ainsi, l'élévation du niveau plasmatique du cortisol peut être mis en relation avec des pathologies telles que des troubles et modifications de l'humeur, une altération des mécanismes de mémorisation entraînant des déficits cognitifs. Des expérimentations récentes confirment que les concentrations élevées de cortisol provoquent des altérations cérébrales au niveau neuronal. On sait également que le stress serait à l'origine d'une modulation de plusieurs aspects de la réponse cellulaire immunitaire. Ceci serait le résultat d'une perturbation des transferts de signaux entre le système nerveux central et le système immunitaire qui ont lieu en partie grâce au système hormonal.

Enfin, l'anxiété provoquée par le stress a des conséquences notamment sur le sommeil, dont le déficit participe à l'épuisement de l'organisme.

D'une manière générale, en tenant compte de la connaissance actuelle des mécanismes du stress, toute réflexion portant sur la protection de l'organisme contre les effets néfastes dus au stress peut donc s'orienter dans les directions suivantes :
- protéger les cellules et organes des excès de cortisol (neuro-protection);
- renforcer les défenses immunitaires (immuno-stimulation);
- agir sur l'anxiété grâce à un effet calmant.

C'est l'objectif principal de l'invention, qui reconnaît l'importance de ce triple mécanisme, et qui propose à cet effet une composition ayant simultanément un effet calmant, régulateur des hormones du stress et immuno-stimulateur. La composition est caractérisée en ce qu'elle comporte :
- au moins un composant incluant notamment une combinaison d'acides linoléique et linolénique selon une proportion pondérale respective de 58% et 13% ;
- au moins un composant pris dans le groupe des flavonoïdes, consistant en du ginseng, sous la forme de ginsénosides et autres saponines ; et
- au moins un composant ayant des propriétés calmantes, du type contenant des extraits de houblon (Humulus Lupulus) et/ou du tryptophane issu d'une fraction de protéines de lait.

Des recherches récentes ont en effet mis en évidence des propriétés intéressantes des acides linoléiques et alpha-linoléniques sur la régulation des taux de cortisol. Selon ces études, il ne semble pas que la quantité d'acide linoléique et/ou linolénique ait en soi une influence bénéfique, mais plutôt le ratio précité entre ces deux acides. Cet effet bénéfique serait le résultat d'une meilleure bio-disponibilité cérébrale des principes actifs, ledit ratio étant capable de traverser la barrière hémato-encéphalique et d'augmenter la fluidité membranaire (essentielle pour le fonctionnement du système nerveux).

De préférence, le composant comportant la combinaison d'acide linolénique et linoléique est de l'huile de noix. Ce produit naturel, facilement disponible, présente l'avantage de ne pas nécessiter de travaux de préparation ou de synthèse complexes pour l'obtention du ratio ci-dessus.

L'utilisation de noix, ainsi que de tryptophane et de divers fruits est d'ailleurs mentionnée dans le document EP-0 896 797 dans un exemple de composition fermentée permettant d'améliorer la qualité nutritionnelle de la nourriture avec laquelle cette composition est utilisée et de soulager le stress d'organismes végétaux et animaux traités avec ce type de composition.

On sait par ailleurs depuis longtemps que le flavonoïde choisi, le ginseng comportant des ginsénosides et autres saponines, améliore l'endurance physique et les capacités mentales à la fois chez l'homme et l'animal. Par exemple, les propriétés toniques du ginseng ont été étudiées sur différents paramètres tels que l'appétit, le sommeil, l'absence de mouvements d'humeur, l'efficacité au travail,... etc. Le ginseng régule également les concentrations en corticostéroïdes, des études chez l'animal ayant montré que le ginseng diminuait les concentrations sanguines de cortisol. D'autres études ont montré son action immunostimulante. Ainsi, ces flavonoïdes possèdent en eux-mêmes à la fois un effet calmant, régulateur des hormones du stress et immuno-stimulateur.

L'utilisation de flavonoïdes, de tryptophane et d'une combinaison d'acides linoléique et linolénique a déjà été décrite, dans le document EP-0 147 699, mais pour une application différente, à savoir le traitement de la lèpre, et avec des composants différents. Le ratio d'acides résulte en effet de l'utilisation d'huile de soja, alors que les flavonoïdes sont contenus dans de la poudre de cacao.

Enfin, les extraits de houblon (Humulus Lupulus) présentent une action calmante et sédative particulièrement intéressante. Des études ont montré que ces extraits ont une activité antispasmodique sur différentes préparations de muscles lisses isolés et des propriétés sédatives conduisant à une amélioration de l'aptitude à faire face au stress et à la nervosité.

La composition de l'invention peut également contenir, comme composant ayant des propriétés sédatives, du tryptophane. L'utilisation de tryptophane résulte de l'observation des effets du 5-hydroxytryptophane (synthétisé dans l'organisme à partir du tryptophane) qui est un des proches précurseurs de la sérotonine. Celle-ci, présente au niveau du cerveau, des plaquettes sanguines et de l'appareil gastro-intestinal, permet la régulation de l'humeur, du comportement et du cycle du sommeil. Ainsi, le tryptophane et son métabolite sont en théorie capables d'apporter des effets thérapeutiques similaires, sans les effets secondaires. On sait en particulier que les personnes souffrant d'insomnies chroniques répondent bien au tryptophane lorsque ce dernier est administré à faible dose de manière répétée.

Le tryptophane utilisé dans l'invention est apporté par une fraction de protéines de lait et représente un pourcentage pondéral de l'ordre de 3% de cette fraction.

Les différents éléments participant à la composition de l'invention permettent ainsi d'aboutir au triple résultat recherché, les différents constituants agissant en combinaison et renforçant mutuellement leurs effets.

Selon une possibilité, la composition de l'invention comprend également de l'extrait d'althée (guimauve), contenant entre autres une substance ayant un effet immunostimulant (arabinogalactane).

La composition de l'invention doit bien entendu être dosée pour une prise périodique calculée afin que le sujet puisse en ressentir les effets bénéfiques par obtention du triple effet précité. Ainsi, pour un usage quotidien la composition de l'invention comprend les proportions pondérales suivantes :
- 600 à 4000 mg d'huile de noix ;
- extrait de ginseng tel que la quantité en ginsenosides soit comprise entre 1 et 30 mg ;
- 100 à 400 mg d'extrait de houblon ES ;
- 40 à 1000 mg de fraction de protéines de lait.

Selon un dosage préférentiel, la composition est constituée de :
- 720 mg d'huile de noix ;
- 13 mg d'extrait de ginseng tel que la quantité en ginsenosides soit égale à 1,6 mg;
- 150 mg d'extrait de houblon ES ;
- 40 mg de fraction de protéines de lait.

De plus, selon une possibilité, l'extrait d'althée est présent à hauteur de 10 mg.

Afin de faciliter l'absorption par le corps de ladite composition, différents conditionnements et véhicules sont proposés :
- gélules et capsules ;
- ampoules ;
- microbilles ;
- produits de confiserie (par exemple des chocolats) ;
- préparations pour boissons froides ou chaudes (par exemple des tisanes).

Des exemples précis en sont donnés dans la suite.

### 1. Conditionnement par capsule

Chaque capsule est par exemple formée d'une enveloppe à base de gélatine, glycérol, amidon et de colorants, ladite enveloppe contenant :
- 360 mg d'huile de noix ;
- 5 mg d'extrait d'althée ;
- 6,5 mg d'extrait de ginseng ;
- 75 mg d'extrait de houblon ;
- 20 mg de protéines de lait riches en tryptophanes ;
et, comme excipients et agents technologiques :
- de la cire jaune d'abeille ;
- de la lécithine de soja à raison d'environ 70 mg.

### 2. Conditionnement par ampoule

Selon la même logique, une ampoule contient à titre d'exemple :
- 720 mg d'huile de noix ;
- 10 mg d'extrait d'althée ;
- 13 mg d'extrait de ginseng ;
- 150 mg d'extrait de houblon ;
- 40 mg de protéines de lait riches en tryptophanes ;
et, comme excipients et agents technologiques :
- du jus de fruit ;
- des ovophospholipides ; et
- des produits antioxydants.

### 3. Intégration à un chocolat

Enfin, un chocolat anti-stress peut être constitué de la manière suivante :
- sucre ;
- pâte de cacao ;
- beurre de cacao ;
- poudre de noix en quantité correspondant à 720 mg d'huile de noix ;
- 10 mg d'extrait d'althée ;
- 13 mg d'extrait de ginseng ;
- 150 mg d'extrait de houblon ;
- 40 mg de protéines de lait riches en tryptophanes ;
et, comme agents technologiques :
- de la lécithine de soja ; et
- des arômes.

Selon une variante, la composition de l'invention peut même être incorporée dans des huiles essentielles.

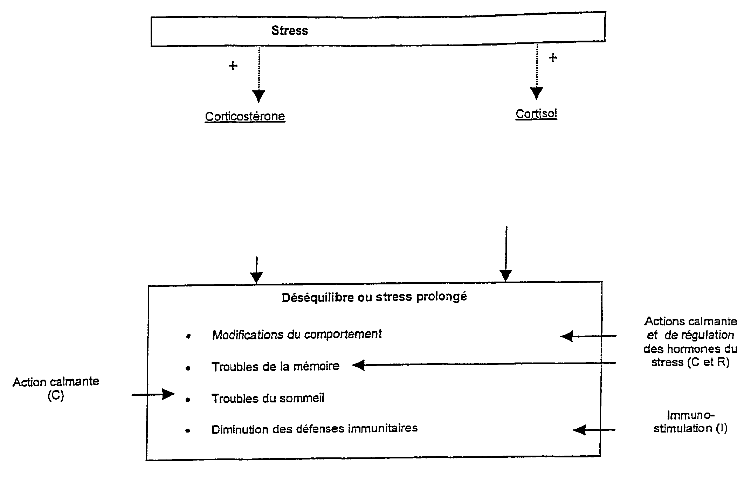

L'invention sera mieux comprise en référence à la figure 1, qui comporte un diagramme résumant d'une part les effets du stress sur l'organisme humain, ainsi que la nécessité de répondre à ces effets par trois types d'actions réalisés par la composition de l'invention. En somme, le stress peut être engendré lors de situations quotidiennes vécues par les patients, comme la peur, la douleur, une hypoglycémie engendrant comme mentionné auparavant une augmentation des taux des hormones du stress (notamment cortisol) dans le sang. Dans le cas où le stress se prolonge, le comportement du patient se modifie (fuite, agressivité, dépression) et ce dernier ressent en particulier des troubles de la mémoire et du sommeil. Enfin, une situation de stress prolongé conduit à une diminution des défenses immunitaires, et par conséquent à une plus grande sensibilité aux infections.

Pour répondre aux effets néfastes d'une situation de stress et/ou troubles de la mémoire, le produit de l'invention exerce une action calmante (C) et de régulation des hormones du stress (R). Pour répondre aux troubles du sommeil, ladite composition exerce une action calmante (C). Enfin, la dernière action d'immuno-stimulation (I) permet de palier la diminution des défenses immunitaires.

La présente invention a été décrite au moyen d'un exemple de composition qui n'est nullement limitatif de l'invention. Celle-ci englobe au contraire les variantes qui sont à la portée de l'homme de l'art.

## Revendications

1. Composition anti-stress pour utilisation dans des véhicules nutritionnels ou plus généralement assimilables par l'organisme humain, dans le but de lui procurer un triple effet calmant, régulateur des hormones du stress et immuno-stimulateur, comportant :
- au moins un composant incluant notamment une combinaison d'acides linoléique et linolénique combinés selon une proportion pondérale respective de 58% et 13% ;
- au moins un composant pris dans le groupe des flavonoïdes, consistant en du ginseng, sous la forme de ginsénosides et autres saponines ; et
- au moins un composant ayant des propriétés calmantes, du type contenant des extraits de houblon (humulus lupulus) et/ou du tryptophane issu d'une fraction de protéines de lait.

2. Composition anti-stress selon la revendication précédente, **caractérisée en ce que** le composant comportant la combinaison d'acides linolénique et linoléique est de l'huile de noix.

3. Composition anti-stress selon l'une des revendications précédentes, **caractérisée en ce que** le tryptophane de la fraction de protéines de lait représente un pourcentage pondéral de l'ordre de 3% de ladite fraction.

4. Composition anti-stress selon l'une quelconque des revendications précédentes, **caractérisée en ce qu'**elle comprend de l'extrait d'althée.

5. Composition selon l'une des revendications 2 à 4, **caractérisée en ce qu'**elle est dosée pour un usage quotidien selon les proportions pondérales suivantes :
- 600 à 4000 mg d'huile de noix ;
- extrait de ginseng tel que la quantité en ginsenosides soit comprise entre 1 et 30 mg ;
- 100 à 400 mg d'extrait de houblon ES ;
- 40 à 1000 mg de fraction de protéines de lait.

6. Composition selon la revendication précédente, **caractérisée en ce qu'**elle est dosée pour un usage quotidien selon les proportions pondérales suivantes :
- 720 mg d'huile de noix ;
- 13 mg d'extrait de ginseng tel que la quantité en ginsenosides soit égale à 1,6 mg ;
- 150 mg d'extrait de houblon ES ;
- 40 mg de fraction de protéines de lait.

7. Composition selon l'une des revendications 5 et 6, **caractérisée en ce que** l'extrait d'althée est présent à hauteur de 10 mg.

8. Composition selon l'une quelconque des revendications précédentes, **caractérisée en ce qu'**elle est incorporée dans les conditionnements suivants :
- gélules et capsules ;
- ampoules ;
- microbilles ;
- produits de confiserie, dont chocolats ; et
- préparations pour boissons fraîches et chaudes, dont tisanes.

9. Capsule intégrant une composition anti-stress selon l'une quelconque des revendications 4 à 8, **caractérisée en ce qu'**elle est formée d'une enveloppe à base de gélatine, glycérol, amidon et de colorants, ladite enveloppe contenant :
- 360 mg d'huile de noix ;
- 5 mg d'extrait d'althée ;
- 6,5 mg d'extrait de ginseng ;
- 75 mg d'extrait de houblon ;
- 20 mg de protéines de lait riches en tryptophanes ;
et, comme excipients et agents technologiques :
- de la cire jaune d'abeille ;
- de la lécithine de soja à raison d'environ 70 mg.

10. Ampoule intégrant une composition anti-stress selon l'une quelconque des revendications 4 à 8, **caractérisée en ce qu'**elle contient :
- 720 mg d'huile de noix ;
- 10 mg d'extrait d'althée ;
- 13 mg d'extrait de ginseng ;
- 150 mg d'extrait de houblon ;
- 40 mg de protéines de lait riches en tryptophanes ;
et, comme excipients et agents technologiques :
- du jus de fruit ;
- des ovophospholipides ; et
- des produits antioxydants.

11. Chocolat intégrant une composition anti-stress selon l'une quelconque des revendications 4 à 8, **caractérisé en ce qu'**il est constitué de :
- sucre ;
- pâte de cacao ;
- beurre de cacao ;
- poudre de noix en quantité correspondant à 720 mg d'huile de noix ;
- 10 mg d'extrait d'althée ;
- 13 mg d'extrait de ginseng ;
- 150 mg d'extrait de houblon ;
- 40 mg de protéines de lait riches en tryptophanes ;
et, comme agents technologiques :
- de la lécithine de soja ; et
- des arômes.

12. Composition selon l'une quelconque des revendications 1 à 7, **caractérisée en ce qu'**elle est incorporée dans des huiles essentielles.

## Patentansprüche

1. Antistress-Zusammensetzung zur Verwendung in Nahrungsmittel-Trägern oder die allgemein vom menschlichen Organismus assimilierbar ist, mit dem Ziel, eine dreifache Wirkung, nämlich eine Beruhigungswirkung, eine regulierende Wirkung auf Stresshormone und eine immunostimulierende Wirkung, zu erzielen, enthaltend:
- mindestens einen Bestandteil, der in erster Linie eine Kombination aus Linolsäure und Linolensäure in einem Gewichtsanteil von 58 % bzw. 13 % enthält;
- mindestens einen Bestandteil aus der Gruppe der Flavonoide, bestehend aus Ginseng in Form von Ginsenosiden und anderen Saponinen; und
- mindestens einen Bestandteil mit beruhigenden Eigenschaften vom Typ mit einem Gehalt an Extrakten von Hopfen (Humulus lupulus) und/oder Tryptophan, das aus einer Fraktion von Milchproteinen stammt.

2. Antistress-Zusammensetzung nach dem vorstehenden Anspruch, **dadurch gekennzeichnet, dass** es sich bei dem Bestandteil, der die Kombination aus Linolensäure und Linolsäure umfasst, um Nussöl handelt.

3. Zusammensetzung nach einem der vorstehenden Ansprüche, **dadurch gekennzeichnet, dass** das Tryptophan der Fraktion von Milchproteinen einen Gewichtsanteil in der Größenordnung von 3 % dieser Fraktion ausmacht.

4. Antistress-Zusammensetzung nach einem der vorstehenden Ansprüche, **dadurch gekennzeichnet, dass** sie Althee-Extrakt enthält.

5. Zusammensetzung nach einem der Ansprüche 2 bis 4, **dadurch gekennzeichnet, dass** sie zur täglichen Anwendung entsprechend den folgenden Gewichtsverhältnissen dosiert wird:
- 600 bis 4000 mg Nussöl;
- Ginsengextrakt in der Menge, dass die Menge an Ginsenosiden 1 bis 30 mg beträgt;
- 100 bis 400 mg Hopfenextrakt ES;
- 40 bis 1000 mg der Fraktion von Milchproteinen.

6. Zusammensetzung nach dem vorstehenden Anspruch, **dadurch gekennzeichnet, dass** sie zur täglichen Anwendung entsprechend den folgenden Gewichtsverhältnissen dosiert wird:
- 720 mg Nussöl;
- 13 mg Ginsengextrakt, so dass die Menge an Ginsenosiden 1,6 mg beträgt;
- 150 mg Hopfenextrakt ES;
- 40 mg der Fraktion von Milchproteinen.

7. Zusammensetzung nach einem der Ansprüche 5 und 6, **dadurch gekennzeichnet, dass** der Althee-Extrakt in einer Menge von 10 mg vorliegt.

8. Zusammensetzung nach einem der vorstehenden Ansprüche, **dadurch gekennzeichnet, dass** sie in den folgenden Aufmachungen einverleibt ist:
- Gelatinekapseln oder Kapseln;
- Ampullen;
- Mikrokügelchen;
- Süßwaren, darunter Schokoladen; und
- Zubereitungen für kalte und warme Getränke, darunter Tees.

9. Kapsel, enthaltend eine Antistress-Zusammensetzung nach einem der Ansprüche 4 bis 8, **dadurch gekennzeichnet, dass** sie aus einer Hülle auf der Basis von Gelatine, Glycerin, Stärke und farbgebenden Mitteln gebildet ist, wobei die Hülle folgendes enthält:
- 360 mg Nussöl;
- 5 mg Althee-Extrakt;
- 6,5 mg Ginsengextrakt;
- 75 mg Hopfenextrakt;
- 20 mg Milchproteine, die reich an Tryptophanen sind; und
als Exzipientien und technologische Mittel:
- gelbes Bienenwachs;
- Soja-Lecithin in einer Menge von etwa 70 mg.

10. Ampulle enthaltend eine Antistress-Zusammensetzung nach einem der Ansprüche 4 bis 8, **dadurch gekennzeichnet, dass** sie folgendes enthält:
- 720 mg Nussöl;
- 10 mg Althee-Extrakt;
- 13 mg Ginsengextrakt;
- 150 mg Hopfenextrakt;
- 40 mg Milchproteine, die reich an Tryptophanen sind; und
als Exzipientien und technologische Mittel:
- Fruchtsaft;
- Ovophospholipide; und
- Antioxidationsmittel.

11. Schokolade, enthaltend eine Antistress-Zusammensetzung nach einem der Ansprüche 4 bis 8, **dadurch gekennzeichnet, dass** sie besteht aus:
- Zucker;
- Kakaopaste;
- Kakaobutter;
- Nussmehl in einer Menge, die 720 mg Nussöl entspricht;
- 10 mg Althee-Extrakt;
- 13 mg Ginsengextrakt;
- 150 mg Hopfenextrakt;
- 40 mg Milchproteine, die reich an Tryptophanen sind; und
als technologische Mittel:
- Sojalecithin; und
- Aromastoffe.

12. Zusammensetzung nach einem der Ansprüche 1 bis 7, **dadurch gekennzeichnet, dass** sie in essentiellen Ölen einverleibt ist.

## Claims

1. Anti-stress composition for use in nutritional carriers or more generally carriers which can be assimilated by the human organism, with a view to providing it with a triple calming, stress hormone-regulating and immuno-stimulating effect, comprising:
- at least one component incorporating in particular a combination of linoleic and linolenic acids combined in a respective proportion of 58% and 13% by weight;
- at least one component selected from the group of flavonoids, consisting of ginseng in the form of ginsenosides and other saponins; and
- at least one component having calming properties, of the type containing extracts of hops (humulus lupulu) and/or tryptophan from a fraction of milk proteins.

2. Anti-stress composition as claimed in the preceding claim, **characterised in that** the component incorporating the combination of linoleic and linolenic acids is walnut oil.

3. Anti-stress composition as claimed in one of the preceding claims, **characterised in that** the tryptophan from the fraction of milk proteins accounts for a percentage by weight of 3% in said fraction.

4. Anti-stress composition as claimed in any one of the preceding claims, **characterised in that** it contains extract of althea.

5. Composition as claimed in one of claims 2 to 4, **characterised in that** it is administered in a dose intended for daily use based on the following proportions by weight:
- 600 to 4000 mg of walnut oil;
- ginseng extract in an amount such that the quantity of ginsenosides is between 1 and 30 mg;
- 100 to 400 mg of extract of hops ES;
- 40 to 1000 mg of fraction of milk proteins.

6. Composition as claimed in the preceding claim, **characterised in that** it is administered in a dose intended for daily use based on the following proportions by weight:
- 720 mg of walnut oil;
- 13 mg of ginseng extract, such that the quantity of ginsenosides is equal to 1.6 mg;
- 150 mg of extract of hops ES;
- 40 mg of fraction of milk proteins.

7. Composition as claimed in one of claims 5 and 6, **characterised in that** it contains an amount of 10 mg of extract of althea.

8. Composition as claimed in any one of the preceding claims, **characterised in that** it is incorporated in one of the following formats:
- gels and capsules;
- ampoules;
- micro-spheres;
- confectionery products, including chocolates; and
- preparations for cold and hot drinks, including infusions.

9. Capsule integrating an anti-stress composition as claimed in any one of claims 4 to 8, **characterised in that** it is made from a casing with a base of gelatine, glycerine, starch and colourants, said casing containing:
- 360 mg of walnut oil;
- 5 mg of extract of althea;
- 6.5 mg of ginseng extract;
- 75 mg of extract of hops;
- 20 mg of milk proteins with a high tryptophan content;
and, as excipients and technological agents:
- yellow beeswax;
- soya lecithin in a quantity of approximately 70 mg.

10. Ampoule integrating an anti-stress composition as claimed in any one of claims 4 to 8, **characterised in that** it contains:
- 720 mg of walnut oil;
- 10 mg of extract of althea;
- 13 mg of ginseng extract;
- 150 mg of extract of hops;
- 40 mg of milk proteins with a high tryptophan content;
and, as excipients and technological agents:
- fruit juice;
- ovophospholipids; and
- antioxidant products.

11. Chocolate integrating an anti-stress composition as claimed in any one of claims 4 to 8, **characterised in that** it is made from:
- sugar;
- cocoa paste;
- cocoa butter;
- powdered walnut in a quantity corresponding to 720 mg of walnut oil;
- 10 mg of extract of althea;
- 13 mg of ginseng extract;
- 150 mg of extract of hop;
- 40 mg of milk proteins with a high tryptophan content;
and, as technological agents:
- soya lecithin; and
- flavourings.

12. Composition as claimed in any one of claims 1 to 7, **characterised in that** it is incorporated in essential oils.
